# FASCICULE DE BREVET EUROPEEN

(11) **EP 4 525 779 B1**
(45) Date de publication et mention de la délivrance du brevet: **05.08.2026**
(21) Numéro de dépôt: 23730168.4
(22) Date de dépôt: 17.05.2023
(51) Int. Cl.: A61F 2/12

(54) **PROTHÈSE MAMMAIRE IMPLANTABLE ET METHODE DE FABRICATION**
IMPLANTIERBARE BRUSTPROTHESE UND HERSTELLUNGSVERFAHREN
IMPLANTABLE BREAST PROSTHESIS AND MANUFACTURING METHOD

(30) Priorité: 17.05.2022 FR 2204665
(43) Date de publication de la demande: 26.03.2025
(73) Titulaire: Medical Innovation Developpement, 69570 Dardilly (FR)
(72) Inventeur: MOJALLAL, Ali, 69006 Lyon (FR)
(86) Numéro de dépôt international: PCT/FR2023/050713
(87) Numéro de publication internationale: WO 2023/222983

(56) Documents cités:
- US-A1- 2010 114 312
- US-A1- 2011 270 391
- US-A1- 2015 351 900

## Description

### Domaine technique

La présente divulgation se rapporte à une prothèse mammaire et à sa méthode de fabrication.

### Technique antérieure

Il est connu d'avoir recours à des prothèses mammaires pouvant être implantées dans la poitrine d'une patiente pour réaliser une reconstruction mammaire, suite à une ablation ou mastectomie dans le cadre du traitement d'un cancer ou encore suite à une nécrose résultant d'une infection ou d'un traumatisme. Les prothèses mammaires peuvent également être utilisées à des fins esthétiques pour modifier la forme ou le volume de la poitrine.

Il existe différents types de prothèse mammaire qui diffèrent notamment par :
- leur forme, qui peut être par exemple anatomique, ronde, de type prothèse d'expansion ou de type prothèse asymétrique (gauche, droite),
- leur enveloppe extérieure, qui peut être par exemple en polyuréthane ou en silicone,
- par la texturation de leur enveloppe extérieure, qui peut être par exemple lisse, microtexturée ou macrotexturée,
- par le produit de remplissage de l'enveloppe extérieure, qui peut être par exemple du gel de silicone, du sérum physiologique ou de l'hydrogel.

Notamment, le document US 2011/270391 A1 divulgue une prothèse mammaire implantable comprenant un squelette intérieur souple délimitant une chambre de remplissage ainsi qu'une enveloppe extérieure souple délimitant une deuxième chambre de remplissage remplie d'un deuxième fluide.

Outre les risques liés à l'apparition de coque périprothétique, de plis, vagues ou rotation, d'inflammation ou d'infection, les prothèses mammaires connues présentent principalement un risque de rupture ou de dégonflement conduisant à une fuite du produit de remplissage. Lorsqu'il s'agit de gel de silicone, cette fuite constitue un véritable danger pour la patiente, compte tenu de l'inflammation locale et générale, de l"envahissement des ganglions lymphatiques et de la formation de siliconome qui peuvent en résulter.

Il existe donc un besoin pour une prothèse mammaire qui présente réduisant les risques pour la patiente.

Le confort pour la patiente et la restauration mammaire au plus proche des attentes de la patiente notamment en termes d'esthétisme et de toucher, sont par ailleurs des spécifications incontournables.

### Résumé

La présente divulgation vient répondre à ce besoin.

Il est proposé une prothèse mammaire implantable selon la revendication 1 comprenant :
■ un squelette intérieur souple et délimitant une première chambre de remplissage remplie d'un premier fluide, ce squelette intérieur comprenant :
   ◆ une base intérieure présentant des surfaces interne et externe opposées,
   ◆ une coiffe intérieure présentant des surfaces interne et externe et solidarisée à la base intérieure de telle manière que les surfaces internes de la base intérieure et de la coiffe intérieure soient en regard l'une de l'autre,
   ◆ une structure d'entretoisement disposée entre les surfaces internes de la base intérieure et de la coiffe intérieure et configurée pour espacer la coiffe intérieure de la base intérieure, de telle manière que la surface externe de la coiffe intérieure présente une convexité,
■ une enveloppe extérieure souple et délimitant une deuxième chambre de remplissage remplie d'un deuxième fluide, cette deuxième chambre de remplissage entourant le squelette intérieur.

Ainsi, la prothèse mammaire selon l'invention propose une structure d'entretoisement garantissant le maintien d'une forme, d'une tenue et d'un volume quels que soient les premier et deuxième fluides, avec lesquels les première et deuxième chambres de remplissage sont remplies. Selon des dispositions particulières, la structure d'entretoisement permet de s'affranchir de l'utilisation de gel de silicone à l'origine des problèmes majeurs connus.

L'un des atouts de cette prothèse tient également dans sa légèreté.

Un autre point positif à mettre au crédit de cette prothèse est le renvoi d'effort homogène en surface (2^{éme} chambre), lors de la palpation.

Selon des dispositions particulières, la prothèse mammaire selon l'invention permet de diminuer le traumatisme chirurgical lié à sa pose en position rétromusculaire. La prothèse mammaire peut notamment permettre une pose en position prépectorale avec un résultat naturel, évitant ainsi une section partielle ou une désinsertion du muscle pectoral.

La conception de la prothèse mammaire en deux parties, à savoir un squelette intérieur et une enveloppe extérieure, permet également, selon des dispositions particulières, de :
- sécuriser la texture de l'enveloppe extérieure aujourd'hui incriminée dans la physiopathologie des lymphomes anaplasiques, et de réactions inflammatoires chroniques,
- sécuriser la technique de pose, éliminant tout risque de rupture (traumatisme de l'implant) lors de l'implantation,
- sécuriser la position de la prothèse mammaire pour éviter les déplacements et rotations secondaires et les interventions chirurgicales ultérieures pour y remédier ; la prothèse mammaire peut en effet posséder un ou plusieurs éléments de fixation sécurisant sa position, en remplacement des fixations par inflammation périprothétique des prothèses mammaires en polyuréthane.

Avantageusement:
- la base intérieure présente un contour autour d'un axe de base (A);
- et la structure d'entretoisement comporte au moins une paroi d'entretoisement souple présentant des premier et deuxième bords opposés solidarisés, respectivement, aux surfaces internes de la base intérieure et de la coiffe intérieure.

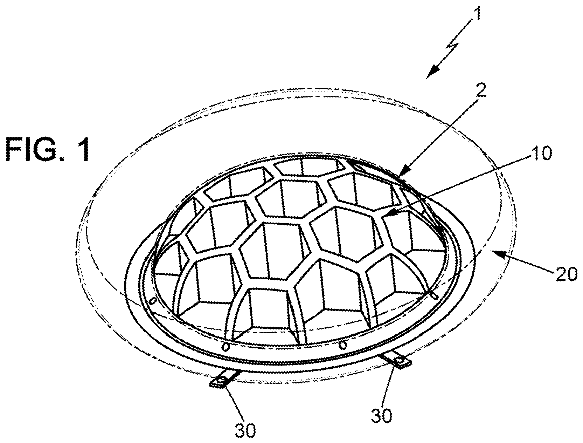

Dans un premier mode de réalisation montré sur les figures 1 à 3, la structure d'entretoisement est une structure en nid d'abeilles (alvéoles de section hexagonale ou circulaire, par exemple).

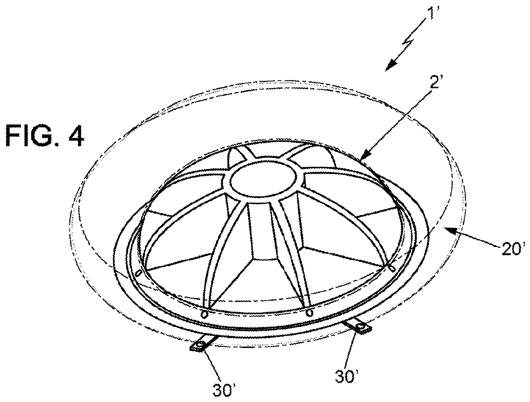

Dans un second mode de réalisation montré sur les figures 4 à 6, le premier bord et le deuxième bord sont alignés selon l'axe de base (A) et le premier bord, de préférence sensiblement perpendiculaire à l'axe de base (A).

Dans un 3^{e} mode de réalisation non représentée sur les figures, la (ou les) paroi(s) de la structure d'entretoisement a (ont) une forme annulaire d'axe (A).

Pour certaines des parois de la structure en nid d'abeilles du premier mode de réalisation et pour au moins une partie des parois, de préférence pour toutes les parois, dans le 2^{e} mode de réalisation, le deuxième bord peut être est aligné avec le premier bord selon l'axe de base (A).

Dans ces 3 modes de réalisation, parmi d'autres, la structure d'entretoisement comporte un agencement de parois d'entretoisement, configuré pour définir une pluralité de compartiments dans la première chambre de remplissage.

Dans certains de ces modes de réalisation, en particulier dans le 1^{er} et le 2^{e} mode de réalisation, les compartiments présentent respectivement des axes (B) de compartiment parallèles à l'axe de base (A).

Selon une variante de réalisation, les parois d'entretoisement de la structure du même nom, peuvent présenter des orifices de communication entre les compartiments de la première chambre de remplissage. Cela permet une certaine circulation du premier fluide de remplissage dans cette première chambre, entre tout ou partie des compartiments.

Selon une caractéristique remarquable de l'invention, la structure d'entretoisement a un sommet centré sur l'axe de base.

Selon des dispositions avantageuses de l'invention,
- l'enveloppe extérieure de la prothèse présente une surface interne orientée vers la deuxième chambre de remplissage et une surface externe opposée à la surface interne;
- le squelette intérieur présente une jupe périphérique solidarisée à la surface interne de l'enveloppe extérieure, de telle manière que la deuxième chambre de remplissage présente:
   ➢ un premier compartiment entre la surface interne de l'enveloppe extérieure, la surface externe de la coiffe intérieure et la jupe périphérique,
   ➢ et un deuxième compartiment entre la surface interne de l'enveloppe extérieure, la surface externe de la base intérieure et la jupe périphérique.

Conformément une caractéristique privilégiée de l'invention, les premier et deuxième compartiments de la deuxième chambre sont en communication de fluide, de préférence, par l'intermédiaire de trous ménagés au travers de la jupe périphérique, ces trous étant plus préférentiellement équirépartis.

Cette caractéristique procure une zone tampon pour les vibrations, ce qui contribue grandement au confort de la prothèse.

De préférence, l'enveloppe extérieure de la prothèse comporte:
➢ une base extérieure présentant une surface interne en regard de la surface externe de la base intérieure,
➢ et une coiffe extérieure présentant des surfaces interne et externe;
la base extérieure et la coiffe extérieure étant de préférence configurées pour que les bases intérieure et extérieure soient parallèles entre elles.

La base extérieure, la jupe périphérique et la base intérieure, délimitent ainsi le 2^{e} compartiment de la 2^{e} chambre de remplissage.

Selon une possibilité, la base extérieure peut être une pièce différente de la coiffe extérieure et être ainsi rapportée et solidarisée, à la coiffe extérieure et à la jupe périphérique, après remplissage des 2 compartiments de la 2^{e} chambre de remplissage, avec le 2^{e} fluide de remplissage.

Le squelette intérieur et l'enveloppe extérieure de la prothèse, sont préférablement réalisés dans un matériau élastomère naturel ou synthétique, de dureté Shore A, de préférence comprise entre 10 et 80, plus préférentiellement encore entre 15 et 50, et mieux encore entre 25 et 35.

Ce matériau élastomère est avantageusement choisi dans le groupe comprenant - idéalement constitué par - les matériaux suivants :
* les silicones, en particulier les silicones "*Liquid Silicon Rubber*" (LSR) ou les silicones "*Heat Cured Rubber* ou *High Consistency Rubber*" (HCR); en particulier des silicones comprenant des polydiméthylsiloxanes (PDMS);
* les plastomères, en particulier les copolymères à blocs polydiméthylsiloxane (PDMS) et polyméthacrylate de méthyle (PMMA) ;
* les copolymères comprenant du PLA (PLA : acide polylactique), en particulier les PLA-b-PEG (PEG : polyéthylène glycol);
* et les mélanges de ces matériaux.

Les premier et deuxième fluides de remplissage peuvent être différents l'un de l'autre, le premier fluide étant gazeux à une température supérieure ou égale à 30°C et à pression atmosphérique, le deuxième fluide étant liquide à une température supérieure ou égale à 30°C et à pression atmosphérique.

Le premier fluide peut comprendre de l'air, de préférence à une pression égale à la pression atmosphérique.

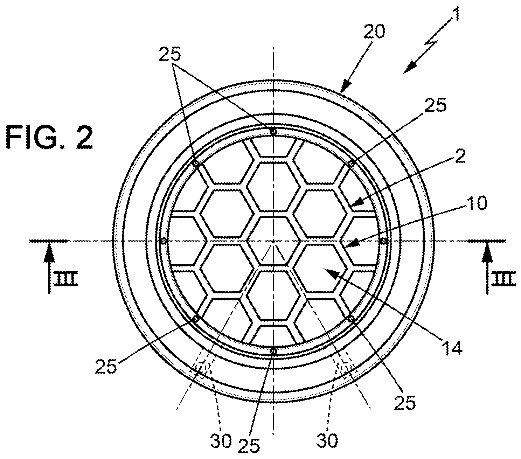

Le deuxième fluide peut comprendre une solution de viscosité comprise entre 100 et 500 Pa.s, de préférence entre 200 et 300 Pa.s. Cette viscosité est mesurée selon la méthode décrite en "2.2.10. *VISCOSITY - ROTATING VISCOMETER METHOD* aux pages 28 & 29 de "*EUROPEAN PHARMACOPEIA 8.0*" en faisant usage d'un "*CONCENTRIC CYLINDER VISCOMETER ABSOLUTE VISCOMETER*"*,* et par mise en oeuvre d'un "*Couette type viscometer*" (fluide non newtonien) tel que montré aux figures 2.2.10.-1 et 2.2.10.-2, à un taux de cisaillement moyen de 1s⁻¹ , variant entre 0,1s⁻¹ et 10s⁻¹, à température et pression atmosphérique ambiantes .

Le deuxième fluide peut comprendre une solution d'acide hyaluronique, de préférence, à une concentration comprise entre 1 % en masse et 5 % en masse, et, plus préférentiellement encore, entre 2 % en masse et 4 % en masse.

L'enveloppe extérieure peut avantageusement comporter au moins un élément de fixation.

Selon un autre aspect, il est proposé selon la revendication 11, un procédé de fabrication de la prothèse mammaire telle que définie précédemment, par moulage et/ou impression 3D de la prothèse en une seule pièce ou des éléments constitutifs de la prothèse en plusieurs pièces, de préférence par moulage et/ou impression 3D du squelette intérieur souple et/ou de l'enveloppe extérieure souple en une seule pièce ou en plusieurs pièces; plus préférentiellement encore par moulage et/ou impression 3D de la structure d'entretoisement et/ou de la coiffe intérieure, et/ou de la jupe périphérique en une seule pièce ou en plusieurs pièces;
ledit procédé comprenant, par ailleurs:
◆ avantageusement, le remplissage de la première et/ou de la deuxième chambre de remplissage, avec le premier et le deuxième fluide de remplissage, respectivement;
◆ et, éventuellement, l'assemblage des éléments constitutifs de la prothèse.

Selon une disposition intéressante de l'invention, tout ou partie des éléments constitutifs de la prothèse définissant au moins l'une des deux chambres de remplissage, sont soumis à un traitement d'étanchéification, de préférence par application d'un revêtement, avantageusement un revêtement polymère, et, mieux encore, un revêtement parylène.

### Brève description des dessins

D'autres caractéristiques, détails et avantages apparaîtront à la lecture de la description détaillée ci-après, et à l'analyse des dessins annexés, sur lesquels :
[Fig. 1] - la figure 1 est une représentation en perspective d'une prothèse mammaire implantable selon un premier mode de réalisation,
[Fig. 2] - la figure 2 est une représentation en vue de dessus de la prothèse mammaire de la figure 1,
[Fig. 3] - la figure 3 est une représentation en coupe selon l'orientation référencée III-III sur la figure 2 de la prothèse mammaire de la figure 1,
[Fig. 4] - la figure 4 est une représentation en perspective d'une prothèse mammaire implantable selon un deuxième mode de réalisation,
[Fig. 5] - la figure 5 est une représentation en vue de dessus de la prothèse mammaire de la figure 4,
[Fig. 6] - la figure 6 est une représentation en coupe selon l'orientation référencée VI-VI sur la figure 5 de la prothèse mammaire de la figure 4.

### Description des modes de réalisation

Les figures 1 à 3 représentent un premier mode de réalisation d'une prothèse mammaire 1 destinée à être implantée dans la poitrine d'une patiente, dans le cadre d'une chirurgie de reconstruction mammaire ou une chirurgie esthétique.

La prothèse mammaire 1 comprend un squelette intérieur 2 souple et une enveloppe extérieure 20 souple, tous deux réalisés en un matériau biocompatible déformable, tel qu'un élastomère silicone de dureté shore A, par exemple égale à 30.

Le matériau est de préférence radio-transparent, afin de ne pas perturber la lecture de mammographies et d'échographies mammaires.

Le squelette intérieur 2 comprend une base intérieure 4 et une coiffe intérieure 5 assemblées l'une à l'autre de manière étanche, pour délimiter une première chambre de remplissage 3. Cette dernière est remplie ou se remplit du premier fluide de remplissage avant assemblage. Selon une variante, le squelette intérieur 2 pourrait être réalisé en une seule pièce, le remplissage de la première chambre avec le premier fluide s'opérant alors de préférence concomitamment à la fabrication du squelette intérieur 2.

La base intérieure 4 présente des surfaces interne 4a et externe 4b opposées et un contour qui, dans le 1^{er} mode de réalisation représenté, s'étend autour d'un axe de base A en étant centré sur l'axe de base A. En particulier, le contour est circulaire.

La coiffe intérieure 5 est solidaire de la base intérieure 4 (par exemple après solidarisation par collage ou soudage), de telle manière qu'une surface interne 5a de la coiffe intérieure 5 soit disposée en regard de la surface interne 4a de la base intérieure 4. En particulier, la surface interne 4a de la base intérieure 4 est solidaire de la surface interne 5a de la coiffe intérieure 5, (par exemple après solidarisation par collage ou soudage), à distance d'un bord périphérique 6 de celle-ci, de telle manière que la coiffe intérieure 5 comporte une jupe périphérique 7 entre son bord périphérique 6 et le contour de la base intérieure 4.

Une structure d'entretoisement 10 est interposée entre les surfaces internes 4a, 5a de la base intérieure 4 et de la coiffe intérieure 5, pour espacer la coiffe intérieure 5 de la base intérieure 4, de telle manière:
□ que la base intérieure 4 puisse être globalement plane lorsqu'elle repose sur une surface d'appui plane, en l'absence de contrainte extérieure,
□ et qu'une surface externe 5b de la coiffe intérieure 5, opposée à la surface interne 5a, présente une convexité.

La structure d'entretoisement 10 comporte un agencement de parois d'entretoisement 11 souples présentant chacune des premier 12 et deuxième 13 bords opposés, solidarisés, respectivement, aux surfaces internes 4a, 5a de la base intérieure 4, et de la coiffe intérieure 5. Afin d'assurer un maintien du squelette intérieur 2 dans la forme voulue, à savoir une base intérieure 4 plane et une coiffe intérieure 5 convexe, le premier bord 12 de chacune des parois d'entretoisement 11 s'étend perpendiculairement à l'axe de base A et le deuxième bord 13 est aligné avec le premier bord 12 selon l'axe de base A. De plus, les deuxièmes bords 13 des parois d'entretoisement 11 présentent des courbures adaptées pour obtenir la convexité de la surface externe 5b de la coiffe intérieure 5. Dans le 1^{er} mode de réalisation représenté, la structure d'entretoisement 10 présente un sommet centré sur l'axe de base A conférant au squelette intérieur 2 une forme de dôme.

Les parois d'entretoisement 11 sont agencées pour définir une pluralité de compartiments 14 présentant respectivement des axes de compartiment B parallèles à l'axe de base A. Dans le premier mode de réalisation représentés sur les figures 1 à 3, l'agencement de parois d'entretoisement 11 forme une structure d'entretoisement 10 tridimensionnelle de type nid d'abeilles, dont les alvéoles ont, dans cet exemple, une forme hexagonale en coupe dans un plan perpendiculaire à l'axe de base A. Ces alvéoles sont des compartiments 14, de préférence, étanches entre eux.

L'enveloppe extérieure 20 de la prothèse 1 comporte une base extérieure 22 et une coiffe extérieure 23 en une seule pièce ou solidarisées l'une à l'autre de manière étanche pour délimiter une deuxième chambre de remplissage 21 entourant le squelette intérieur 2 et remplie du 2^{e} fluide de remplissage.

La jupe périphérique 7 du squelette intérieur 2 est solidarisée à une surface interne 20a de l'enveloppe extérieure 20, de telle manière que la surface externe 4b de la base intérieure 4 soit en regard d'une surface interne 22a de la base extérieure 22. La deuxième chambre de remplissage 21 présente alors un premier compartiment 21a entre une surface interne 23a de la coiffe extérieure 23 de l'enveloppe extérieure 20, la surface externe 5b de la coiffe intérieure 5 et la jupe périphérique 7, et un deuxième compartiment 21b entre la surface interne 22a de la base extérieure 22 de l'enveloppe extérieure 20, la surface externe 4b de la base intérieure 4 et la jupe périphérique 7.

La première chambre de remplissage 3 du squelette intérieur 2 est remplie d'un premier fluide et la deuxième chambre de remplissage 21 de l'enveloppe extérieure 20 est remplie d'un deuxième fluide. Les premier 21a et deuxième 21b compartiments communiquent entre eux par l'intermédiaire de trous 25, par exemple équirépartis, ménagés dans la jupe périphérique 7 du squelette intérieur 2. Le 2^{e} fluide de remplissage peut ainsi circuler entre ces 2 compartiments 21a et 21b. La base extérieure 22 et la coiffe extérieure 23 sont configurées pour que les bases intérieure 4 et extérieure 22 soient parallèles entre elles au repos et lorsque la prothèse remplie des deux fluides est posée sur un support plan. Dans cette position de repos, la surface externe 23b de la coiffe extérieure 23 de l'enveloppe extérieure 20, présente une convexité.

Le premier fluide est gazeux. Il s'agit par exemple d'air, de préférence à une pression égale à la pression atmosphérique.

Le deuxième fluide est, par exemple, une solution aqueuse d'acide hyaluronique à une concentration de l'ordre de 3% en masse.

Pour améliorer le maintien en position de la prothèse mammaire 1, cette dernière peut comporter, de préférence sur l'enveloppe extérieure, un ou plusieurs éléments de fixation, par exemple, sous la forme de pattes de fixation.

La prothèse mammaire 1 ainsi réalisée dispose de propriétés mécaniques, telles qu'une souplesse, une tenue, une résistance et une densité, lui conférant une fermeté satisfaisante sur le plan esthétique et au toucher.

La structure d'entretoisement 10 peut présenter différentes tailles d'alvéoles, en fonction d'une localisation centrale ou périphérique par rapport à l'axe de base A par exemple, pour réaliser un squelette intérieur 2 plus ou moins dense donnant différentes souplesses à la palpation.

La prothèse mammaire 1 est également compressible et déformable de sorte à pouvoir être partiellement écrasée et aplatie pour une implantation par une incision comprise entre 2 cm et 10 cm, de préférence entre 3 cm et 6 cm et, plus préférablement encore, de l'ordre de 4 cm.

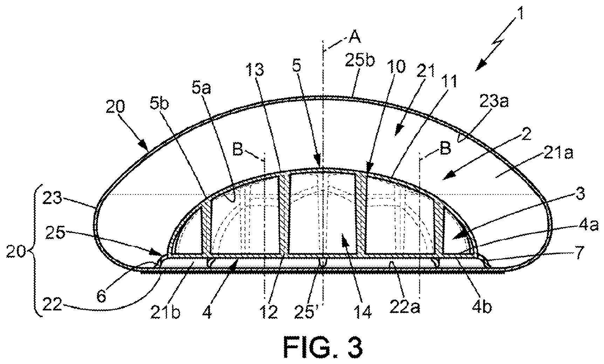

En relation avec les figures 3 à 6, un deuxième mode de réalisation de la prothèse mammaire 1' est décrit.

Dans ce deuxième mode de réalisation, la prothèse mammaire 1' ne diffère du premier mode de réalisation que dans l'agencement des parois d'entretoisement 11' souples de la structure d'entretoisement 10' du squelette intérieur 2'. Les autres caractéristiques, inchangées par rapport au premier mode de réalisation ne sont pas décrites à nouveau ici. On se réfèrera donc à la description qui en a été faite précédemment pour plus de détails.

Des parois d'entretoisement 11', au nombre de huit sur les figures, s'étendent radialement par rapport à l'axe de base A, depuis un tronc creux cylindrique 26', centré sur l'axe de base A. Cet agencement définit un compartiment 14' central et huit compartiments 14' périphériques autour du compartiment 14' central.

Selon une variante, le tronc creux cylindrique 26' peut être remplacé par un tronc plein de diamètre variable. Le diamètre minimum peut correspondre à la dimension de la réunion des parois 11' autour de l'axe de base A.

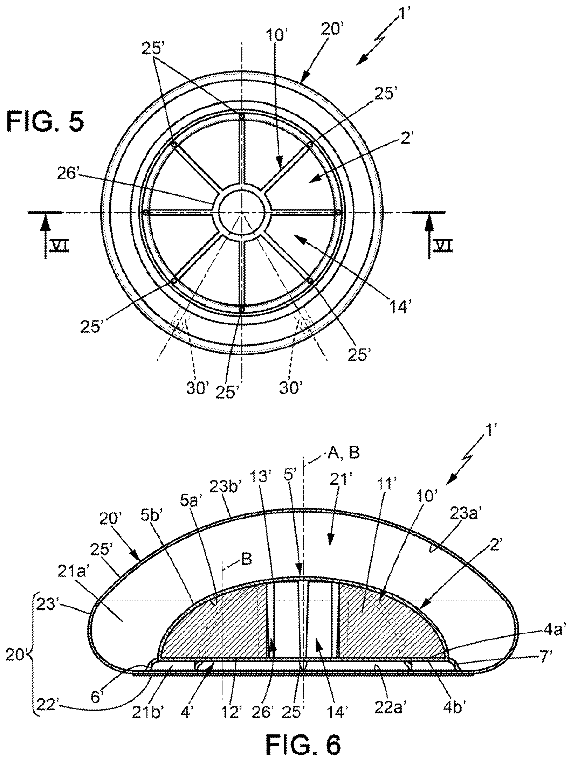

Suivant une caractéristique préférée, la prothèse mammaire 1,1' comprend deux pattes de fixation 30,30' présentant chacune un oeillet permettant la fixation, e.g. par couture, de la prothèse, après implantation. Comme cela apparaît sur les figures 2 et 5, les deux pattes de fixation 30,30' sont symétriques par rapport au plan diamétral sagittal de la prothèse, destiné à être sensiblement parallèle au plan sagittal de la patiente après implantation de la prothèse 1,1'. L'angle formé par chaque patte de fixation 30,30' par rapport au plan diamétral sagittal de la prothèse est, de préférence, compris entre 45 et 15 degrés, et, plus préférentiellement encore, de l'ordre de 30°.

En variante, tout autre agencement d'une ou plusieurs parois d'entretoisement 11,11' souples espaçant la coiffe intérieure 5 de la base intérieure 4 en conférant une convexité à la coiffe intérieure 5 pourrait être prévu.

Ainsi, les parois d'entretoisement pourraient être un ou plusieurs anneaux, notamment circulaires, ovales ou elliptiques, continus ou discontinus, concentrique(s) à l'axe A ou non, ou toutes autres formes de parois.

S'agissant du procédé de fabrication de la prothèse mammaire 1,1', il renvoie aux technologies connues de moulage ou d'impression 3D, à partir d'élastomères, par exemple silicones de dureté Shore A de l'ordre de 30 après réticulation/durcissement. Par moulage on entend notamment, moulage par injection, pressage, emboutissage...

Les différents éléments constitutifs de la prothèse mammaire 1,1' peuvent être manufacturés séparément, en un seul bloc ou en plusieurs sous unités.

Dès lors que la prothèse mammaire 1,1' n'est pas réalisée en un seul bloc, le procédé de fabrication comprend des opérations d'assemblage des différents éléments constitutifs ou sous unités, par soudage et/ou collage.

Pour rendre étanche ou pour améliorer l'étanchéité des différentes parois séparant les compartiments 14,14', 21a ,21a', 21b, 21b', un traitement de revêtement pour un polymère par exemple de parylène est mis en oeuvre.

La fabrication de la prothèse 1,1' intègre, de préférence, le remplissage de la première chambre de remplissage par un 1^{er} fluide gazeux, tel que l'air à pression atmosphérique et le remplissage de la 2^{e} chambre de remplissage constituée par les 2 compartiments 21a ,21a', 21b, 21b', ceignant la coiffe intérieure 5,5', par un 2^{e} fluide formé par un liquide, à savoir par exemple une solution aqueuse d'acide hyaluronique à 2 ou 3 % en masse.

## Revendications

1. Prothèse mammaire (1; 1') implantable comprenant:
- un squelette intérieur (2; 2') souple et délimitant une première chambre de remplissage (3) remplie d'un premier fluide, ce squelette intérieur (2; 2') comprenant :
◆ une base intérieure (4) présentant des surfaces interne (4a) et externe (4b) opposées,
◆ une coiffe intérieure (5) présentant des surfaces interne (5a) et externe (5b) et solidarisée à la base intérieure (4) de telle manière que les surfaces internes (4a; 5a) de la base intérieure (4) et de la coiffe intérieure (5) soient en regard l'une de l'autre,
◆ une structure d'entretoisement (10; 10') disposée entre les surfaces internes (4a, 5a) de la base intérieure (4) et de la coiffe intérieure (5) et configurée pour espacer la coiffe intérieure (5) de la base intérieure (4), de telle manière que la surface externe (5b) de la coiffe intérieure (5) présente une convexité,
- une enveloppe extérieure (20,20') souple et délimitant une deuxième chambre de remplissage (21,21') remplie d'un deuxième fluide, cette deuxième chambre de remplissage (21,21') entourant le squelette intérieur (2; 2').

2. Prothèse mammaire (1; 1') selon la revendication 1, dans laquelle:
- la base intérieure (4,4') présente un contour autour d'un axe de base (A);
- et la structure d'entretoisement (10; 10') comporte au moins une paroi d'entretoisement (11,11') souple présentant des premier (12,12') et deuxième (13,13') bords opposés solidarisés, respectivement, aux surfaces internes (4a,4a',5a,5a') de la base intérieure (4,4') et de la coiffe intérieure (5,5').

3. Prothèse mammaire (1;1') selon la revendication 1 ou 2, dans laquelle la structure d'entretoisement (10;10') comporte un agencement de parois d'entretoisement (11;11') configuré pour définir une pluralité de compartiments (14;14') dans la première chambre de remplissage (3).

4. Prothèse mammaire (1 ; 1') selon l'une quelconque des revendications 1 à 3, dans laquelle la structure d'entretoisement (10 ; 10') présente un sommet centré sur l'axe de base (A).

5. Prothèse mammaire (1 ; 1') selon l'une quelconque des revendications 1 à 4, dans laquelle
- l'enveloppe extérieure (20,20') présente une surface interne orientée vers la deuxième chambre de remplissage (21,21') et une surface externe opposée à la surface interne;
- le squelette intérieur (2;2') présente une jupe périphérique (7,7') solidarisée à la surface interne de l'enveloppe extérieure (20,20'), de telle manière que la deuxième chambre de remplissage (21,21') présente:
➢ un premier compartiment (21a,21a') entre la surface interne de l'enveloppe extérieure (20,20'), la surface externe (5b,5b') de la coiffe intérieure (5,5') et la jupe périphérique (7,7'),
➢ et un deuxième compartiment (21b,21b') entre la surface interne de l'enveloppe extérieure (20,20'), la surface externe (4b,4b') de la base intérieure (4,4') et la jupe périphérique (7,7').

6. Prothèse mammaire (1;1') selon la revendication 5, dans laquelle les premier (21a,21a';21b,21b') et deuxième compartiments de la 2^{e} chambre de remplissage, sont en communication de fluide, de préférence par l'intermédiaire de trous (25, 25') ménagés au travers de la jupe périphérique (7,7'), ces trous (25, 25') étant plus préférentiellement équirépartis.

7. Prothèse mammaire (1;1') selon l'une au moins des revendications précédentes **caractérisée en ce qu'**elle comporte, de préférence sur l'enveloppe extérieure un ou plusieurs éléments ou pattes de fixation.

8. Prothèse mammaire (1;1) selon l'une quelconque des revendications 1 à 7, dans laquelle les premier et deuxième fluides sont différents l'un de l'autre, le premier fluide étant gazeux à une température supérieure ou égale à 30°C et à pression atmosphérique, le deuxième fluide étant liquide à une température supérieure ou égale à 30°C et à pression atmosphérique.

9. Prothèse mammaire (1;1') selon la revendication 8, dans laquelle le premier fluide comprend de l'air, de préférence à une pression égale à la pression atmosphérique.

10. Prothèse mammaire (1;1') selon la revendication 8 et/ou 9, dans laquelle le deuxième fluide est une solution d'acide hyaluronique, de préférence, à une concentration comprise entre 1 % en masse et 5 % en masse, et, plus préférentiellement encore, entre 2 % en masse et 4 % en masse.

11. Procédé de fabrication de la prothèse mammaire (1;1') selon l'une quelconque des revendications 1 à 10, par moulage et/ou impression 3D de la prothèse en une seule pièce ou des éléments constitutifs de la prothèse en plusieurs pièces, de préférence par moulage et/ou impression 3D du squelette intérieur (2; 2') souple et/ou de l'enveloppe extérieure (20) souple en une seule pièce ou en plusieurs pièces; plus préférentiellement encore par moulage et/ou impression 3D de la structure d'entretoisement (10;10') et/ou de la coiffe intérieure (5,5'), et/ou de la jupe périphérique (7,7') en une seule pièce ou en plusieurs pièces;
ledit procédé comprenant, par ailleurs:
◆ avantageusement, le remplissage de la première (3,3') et/ou de la deuxième chambre (21,21') de remplissage, avec le premier et le deuxième fluide de remplissage, respectivement;
◆ et, éventuellement, l'assemblage des éléments constitutifs de la prothèse (1,1').

## Patentansprüche

1. Implantierbare Brustprothese (1; 1'), umfassend:
- ein flexibles inneres Gerüst (2; 2'), das eine erste Füllkammer (3) begrenzt, die mit einem ersten Fluid gefüllt ist, wobei das innere Gerüst (2; 2') umfasst:
• eine innere Basis (4) mit einander gegenüberliegenden inneren (4a) und äußeren (4b) Oberflächen,
• eine innere Haube (5) mit inneren (5a) und äußeren (5b) Oberflächen, die mit der inneren Basis (4) derart verbunden ist, dass die inneren Oberflächen (4a; 5a) der inneren Basis (4) und der inneren Haube (5) einander gegenüberliegen,
• eine Abstandshalterstruktur (10; 10'), die zwischen den inneren Oberflächen (4a, 5a) der inneren Basis (4) und der inneren Haube (5) angeordnet und dazu ausgebildet ist, die innere Haube (5) von der inneren Basis (4) zu beabstanden, derart, dass die äußere Oberfläche (5b) der inneren Haube (5) eine Konvexität aufweist,
- eine flexible äußere Hülle (20, 20'), die eine zweite Füllkammer (21, 21') begrenzt, die mit einem zweiten Fluid gefüllt ist, wobei diese zweite Füllkammer (21, 21') das innere Gerüst (2; 2') umgibt.

2. Brustprothese (1; 1') nach Anspruch 1, wobei:
- die innere Basis (4, 4') eine Kontur um eine Basisachse (A) aufweist;
- und die Abstandshalterstruktur (10; 10') mindestens eine flexible Abstandswand (11, 11') umfasst, die erste (12, 12') und zweite (13, 13') gegenüberliegende Ränder aufweist, welche jeweils mit den inneren Oberflächen (4a, 4a', 5a, 5a') der inneren Basis (4, 4') und der inneren Haube (5, 5') verbunden sind.

3. Brustprothese (1; 1') nach Anspruch 1 oder 2, wobei die Abstandshalterstruktur (10; 10') eine Anordnung von Abstandswänden (11; 11') umfasst, die dazu ausgebildet ist, eine Mehrzahl von Abteilen (14; 14') in der ersten Füllkammer (3) zu definieren.

4. Brustprothese (1; 1') nach einem der Ansprüche 1 bis 3, wobei die Abstandshalterstruktur (10; 10') einen auf der Basisachse (A) zentrierten Scheitel aufweist.

5. Brustprothese (1; 1') nach einem der Ansprüche 1 bis 4, wobei
- die äußere Hülle (20, 20') eine zur zweiten Füllkammer (21, 21') hin orientierte innere Oberfläche und eine der inneren Oberfläche gegenüberliegende äußere Oberfläche aufweist;
- das innere Gerüst (2; 2') eine Umfangsschürze (7, 7') aufweist, die mit der inneren Oberfläche der äußeren Hülle (20, 20') verbunden ist, derart, dass die zweite Füllkammer (21, 21') aufweist:
> ein erstes Abteil (21a, 21a') zwischen der inneren Oberfläche der äußeren Hülle (20, 20'), der äußeren Oberfläche (5b, 5b') der inneren Haube (5, 5') und der Umfangsschürze (7, 7'),
> und ein zweites Abteil (21b, 21b') zwischen der inneren Oberfläche der äußeren Hülle (20, 20'), der äußeren Oberfläche (4b, 4b') der inneren Basis (4, 4') und der Umfangsschürze (7, 7').

6. Brustprothese (1; 1') nach Anspruch 5, wobei das erste (21a, 21a'; 21b, 21b') und das zweite Abteil der zweiten Füllkammer in Fluidverbindung stehen, vorzugsweise über Löcher (25, 25'), die durch die Umfangsschürze (7, 7') hindurch ausgebildet sind, wobei diese Löcher (25, 25') besonders bevorzugt gleichmäßig verteilt sind.

7. Brustprothese (1; 1') nach mindestens einem der vorhergehenden Ansprüche, **dadurch gekennzeichnet, dass** sie vorzugsweise an der äußeren Hülle ein oder mehrere Befestigungselemente oder Befestigungslaschen aufweist.

8. Brustprothese (1; 1') nach einem der Ansprüche 1 bis 7, wobei das erste und das zweite Fluid voneinander verschieden sind, wobei das erste Fluid bei einer Temperatur von größer oder gleich 30 °C und bei atmosphärischem Druck gasförmig ist, und wobei das zweite Fluid bei einer Temperatur von größer oder gleich 30 °C und bei atmosphärischem Druck flüssig ist.

9. Brustprothese (1; 1') nach Anspruch 8, wobei das erste Fluid Luft umfasst, vorzugsweise bei einem Druck, der dem atmosphärischen Druck entspricht.

10. Brustprothese (1; 1') nach Anspruch 8 und/oder 9, wobei das zweite Fluid eine Hyaluronsäurelösung ist, vorzugsweise mit einer Konzentration zwischen 1 Massen-% und 5 Massen-%, und besonders bevorzugt zwischen 2 Massen-% und 4 Massen-%.

11. Verfahren zur Herstellung der Brustprothese (1; 1') nach einem der Ansprüche 1 bis 10, durch Formen und/oder 3D-Druck der Prothese als ein einziges Teil oder der Bestandteile der Prothese als mehrere Teile, vorzugsweise durch Formen und/oder 3D-Druck des flexiblen inneren Gerüsts (2; 2') und/oder der flexiblen äußeren Hülle (20) als ein einziges Teil oder als mehrere Teile; noch bevorzugter durch Formen und/oder 3D-Druck der Abstandshalterstruktur (10; 10') und/oder der inneren Haube (5, 5') und/oder der Umfangsschürze (7, 7') als ein einziges Teil oder als mehrere Teile;
wobei das Verfahren ferner umfasst:
• vorteilhafterweise das Füllen der ersten (3, 3') und/oder der zweiten Füllkammer (21, 21') jeweils mit dem ersten und dem zweiten Füllfluid;
• und gegebenenfalls das Zusammenfügen der Bestandteile der Prothese (1, 1').

## Claims

1. Implantable breast prosthesis (1; 1') comprising:
- a flexible inner skeleton (2; 2') delimiting a first filling chamber (3) filled with a first fluid, said inner skeleton (2; 2') comprising:
• an inner base (4) having opposite inner (4a) and outer (4b) surfaces,
• an inner cap (5) having inner (5a) and outer (5b) surfaces and secured to the inner base (4) in such a way that the inner surfaces (4a; 5a) of the inner base (4) and of the inner cap (5) face one another,
• a spacing structure (10; 10') disposed between the inner surfaces (4a, 5a) of the inner base (4) and of the inner cap (5) and configured to space the inner cap (5) from the inner base (4), in such a way that the outer surface (5b) of the inner cap (5) has a convexity,
- a flexible outer envelope (20, 20') delimiting a second filling chamber (21, 21') filled with a second fluid, said second filling chamber (21, 21') surrounding the inner skeleton (2; 2').

2. Breast prosthesis (1; 1') according to claim 1, wherein:
- the inner base (4, 4') has a contour around a base axis (A);
- and the spacing structure (10; 10') comprises at least one flexible spacing wall (11, 11') having opposite first (12, 12') and second (13, 13') edges secured, respectively, to the inner surfaces (4a, 4a', 5a, 5a') of the inner base (4, 4') and of the inner cap (5, 5').

3. Breast prosthesis (1; 1') according to claim 1 or 2, wherein the spacing structure (10; 10') comprises an arrangement of spacing walls (11; 11') configured to define a plurality of compartments (14; 14') in the first filling chamber (3).

4. Breast prosthesis (1; 1') according to any one of claims 1 to 3, wherein the spacing structure (10; 10') has an apex centred on the base axis (A).

5. Breast prosthesis (1; 1') according to any one of claims 1 to 4, wherein:
- the outer envelope (20, 20') has an inner surface oriented towards the second filling chamber (21, 21') and an outer surface opposite the inner surface;
- the inner skeleton (2; 2') has a peripheral skirt (7, 7') secured to the inner surface of the outer envelope (20, 20'), in such a way that the second filling chamber (21, 21') comprises:
> a first compartment (21a, 21a') between the inner surface of the outer envelope (20, 20'), the outer surface (5b, 5b') of the inner cap (5, 5') and the peripheral skirt (7, 7'),
> and a second compartment (21b, 21b') between the inner surface of the outer envelope (20, 20'), the outer surface (4b, 4b') of the inner base (4, 4') and the peripheral skirt (7, 7').

6. Breast prosthesis (1; 1') according to claim 5, wherein the first (21a, 21a') and second (21b, 21b') compartments of the second filling chamber are in fluid communication, preferably by means of holes (25, 25') formed through the peripheral skirt (7, 7'), said holes (25, 25') being more preferably evenly distributed.

7. Breast prosthesis (1; 1') according to at least one of the preceding claims, **characterised in that** it comprises, preferably on the outer envelope, one or more fastening elements or tabs.

8. Breast prosthesis (1; 1') according to any one of claims 1 to 7, wherein the first and second fluids are different from one another, the first fluid being gaseous at a temperature greater than or equal to 30°C and at atmospheric pressure, the second fluid being liquid at a temperature greater than or equal to 30°C and at atmospheric pressure.

9. Breast prosthesis (1; 1') according to claim 8, wherein the first fluid comprises air, preferably at a pressure equal to atmospheric pressure.

10. Breast prosthesis (1; 1') according to claim 8 and/or 9, wherein the second fluid is a hyaluronic acid solution, preferably at a concentration comprised between 1% by mass and 5% by mass, and still more preferably between 2% by mass and 4% by mass.

11. Method for manufacturing the breast prosthesis (1; 1') according to any one of claims 1 to 10, by moulding and/or 3D printing the prosthesis in a single piece or the constituent elements of the prosthesis in several pieces, preferably by moulding and/or 3D printing the flexible inner skeleton (2; 2') and/or the flexible outer envelope (20) in a single piece or in several pieces; still more preferably by moulding and/or 3D printing the spacing structure (10; 10') and/or the inner cap (5, 5'), and/or the peripheral skirt (7, 7') in a single piece or in several pieces;
said method further comprising:
• advantageously, filling the first (3, 3') and/or the second filling chamber (21, 21') with the first and the second filling fluid, respectively;
• and, optionally, assembling the constituent elements of the prosthesis (1, 1').
